# EUROPÄISCHE PATENTSCHRIFT

(11) **EP 2 014 246 B1**
(45) Veröffentlichungstag und Bekanntmachung des Hinweises auf die Patenterteilung: **12.10.2011**
(21) Anmeldenummer: 08011822.7
(22) Anmeldetag: 01.07.2008
(51) Int. Cl.: A61B 17/28, A61B 17/29

(54) **Medizinisches Instrument**
Medical instrument
Instrument médical

(30) Priorität: 13.07.2007 DE 102007034577
(43) Veröffentlichungstag der Anmeldung: 14.01.2009
(73) Patentinhaber: Karl Storz GmbH & Co. KG, 78532 Tuttlingen (DE)
(72) Erfinder: Bacher, Uwe, 78532 Tuttlingen (DE)
(74) Vertreter: Heuckeroth, Volker

(56) Entgegenhaltungen:
- EP-A1- 0 647 433
- WO-A1-94/20037
- WO-A1-96/24296
- DE-C1- 19 740 847

## Beschreibung

Die Erfindung betrifft ein medizinisches Instrument, mit einem Schaft, der einen Außenschaft und einen Innenschaft aufweist, wobei der Außenschaft den Innenschaft umgibt, mit einem Stellglied zum Drehen des Innenschafts um eine Längsachse relativ zum Außenschaft, wobei das Stellglied an einem proximalen Endbereich des Schafts angeordnet ist, und mit zumindest einem ersten Mitnehmerelement, das mit dem Innenschaft in Eingriff steht, um eine Drehbewegung des Stellglieds auf den Innenschaft zu übertragen.

Ein derartiges medizinisches Instrument ist aus CA 2 212 768 A bekannt.

Medizinische Instrumente der eingangs genannten Art werden beispielsweise in der minimal-invasiven Chirurgie während eines laparoskopischen Eingriffs verwendet, um krankhaftes Gewebe abzutragen oder auch um zuvor geschnittenes Gewebe zu veröden. Dazu wird ein distaler Endbereich des Schafts des medizinischen Instruments in eine Körperöffnung eines Patienten eingeführt, an dem entsprechende Greifwerkzeuge, Elektroden o. Ä. angeordnet sind. Der Schaft des medizinischen Instruments ist üblicherweise zweiteilig ausgebildet und weist einen um die Längsachse des medizinischen Instruments drehbaren Innenschaft auf, der in einem Außenschaft aufgenommen ist. Die Operationswerkzeuge sind hierbei am distalen Endbereich des Innenschafts angeordnet. Die Positionierung der Operationswerkzeuge in einer für den Eingriff günstigen Stellung erfolgt erst nach Einführen des Schafts in das Operationsgebiet durch Drehen des Innenschafts relativ zum Außenschaft um die Längsachse des medizinischen Instruments.

Das aus CA 2 212 768 A bekannte medizinische Instrument ist ein Morcellator und weist einen Außenschaft auf, der einen relativ zu ihm drehbaren Innenschaft umgibt und drehfest mit einem proximalen Ende des Handgriffs des medizinischen Instruments verbunden ist. Der Außenschaft erstreckt sich bis zu einem distalen Ende des Handgriffs, während der Innenschaft durch den Handgriff hindurch bis zu einem proximalen Ende des Handgriffs verläuft. Ein im Handgriff aufgenommener Motor steht über ein Mitnehmerelement mit dem Innenschaft in drehfester Verbindung. Das Mitnehmerelement weist einen Zahnradantrieb auf, der mit einem Bereich des Innenschafts, der proximal des proximalen Endes des Außenschafts angeordnet ist, drehfest verbunden ist, um den Innenschaft um die Längsachse zu drehen.

Es sind ferner medizinische Instrumente allgemein bekannt, die nur einen Außenschaft aufweisen, der mit einem Handgriff drehfest verbunden ist. Der Außenschaft des bekannten Instruments erstreckt sich dabei in das Innere des Handgriffs hinein, und sein proximaler Endbereich ist im Handgriffinneren über eine Mutter mit dem Handgriff verschraubt. Der Handgriff des medizinischen Instruments kann beispielsweise ein bereits vorhandener, üblicherweise für operative Eingriffe im Bereich der minimal-invasiven Chirurgie verwendbarer Handgriff sein.

Möchte man ein solches medizinisches Instrument dahingehend weiterentwickeln, dass es zusätzlich zu dem Außenschaft einen um die Längsachse drehbaren Innenschaft innerhalb des Außenschafts aufweist, ergibt sich die technische Problemstellung, wie eine entsprechende Drehübertragungsmechanik des Instruments ausgestaltet sein müßte, die bei gleichzeitiger Verschraubung des Außenschafts mit dem Handgriff innerhalb des Handgriffs eine Relativdrehung des Innen- und Außenschafts erlaubt.

Eine denkbare Ausgestaltung eines solchen weiterentwickelten medizinischen Instruments beruht auf der Anordnung von Magneten am Stellglied und im proximalen Endbereich des Innenschafts, wodurch die Übertragung der Drehbewegung des Stellglieds auf den Innenschaft berührungslos durch den proximalen Endbereich des Außenschafts hindurch erfolgt.

Allerdings ergibt sich bei dieser Ausgestaltung der Drehübertragungsmechanik der Nachteil, das diese Art der Drehübertragung nur einen geringen Kraftübertrag auf den Innenschaft bereitstellt.

Bei einer weiteren Ausgestaltung des weiterentwickelten Instruments könnte der proximale Endbereich des Außenschafts, der innerhalb des Handgriffs angeordnet ist, vollumfänglich in zwei Teile unterbrochen sein, und das Stellglied könnte im Bereich dieser Unterbrechung zwischen den zwei Teilen des Außenschafts direkt am Innenschaft angreifen.

Bei dieser Ausführungsform des medizinischen Instruments ist es allerdings nachteilig, dass beide Teile des proximalen Endbereichs des Außenschafts drehfest mit dem Handgriff verbunden werden müssen.

Eine noch weitere Ausgestaltung des weiterentwickelten medizinischen Instruments könnte darauf basieren, den Innenschaft vollständig durch den Handgriff hindurch zu führen und das Stellglied proximalseitig außerhalb des Handgriffs direkt am Innenschaft anzuordnen. Der Außenschaft wäre hierbei im Handgriffinneren aufgenommen und drehfest mit dem Handgriff verbunden.

Diese Ausgestaltung des medizinischen Instruments weist jedoch den Nachteil auf, dass das medizinischen Instrument nicht einhändig von einem Benutzer bedient werden kann, da das Stellglied proximalseitig des Handgriffs angeordnet ist.

Es ist daher eine Aufgabe der vorliegenden Erfindung, ein medizinisches Instrument der eingangs genannten Art bereitzustellen, das eine Drehübertragungsmechanik aufweist, die durch den proximalen Endbereich des Außenschafts hindurch auf den Innenschaft wirkt, um eine Drehbewegung des Stellglieds auf den Innenschaft zuverlässig zu übertragen.

Erfindungsgemäß wird die Aufgabe hinsichtlich des eingangs genannten medizinischen Instruments dadurch gelöst, dass das medizinische Instrument zumindest zwei Mitnehmerelemente aufweist, die abwechselnd mit dem Innenschaft in Eingriff stehen, um die Drehbewegung des Stellglieds auf den Innenschaft zu übertragen, wobei die Mitnehmerelemente durch umfänglich begrenzte und zueinander in der Längsrichtung versetzte Öffnungen in einem proximalen Endbereich des Außenschafts hindurchreichen.

Das erfindungsgemäße medizinische Instrument weist einen Außenschaft auf, dessen proximaler Endbereich durch umfänglich begrenzte, axial voneinander beabstandete Öffnungen unterbrochen ist, durch die zumindest zwei Mitnehmerelemente hindurch reichen. Die Mitnehmerelemente stehen abwechselnd mit dem Innenschaft in Eingriff, um eine Drehbewegung des Stellglieds auf den Innenschaft zu übertragen, so dass der Innenschaft relativ zum Außenschaft drehbar ist. Erfindungsgemäß ist unter einem "abwechselnden In-Eingriff-Stehen der Mitnehmerelemente mit dem Innenschaft" zu verstehen, dass die Drehung des Stellglieds auf den Innenschaft durch jeweils ein Mitnehmerelement vermittelt wird, jedoch zum Zeitpunkt des Übergangs zwischen einer Drehübertragung von zwei verschiedenen Mitnehmerelementen beide Mitnehmerelemente mit dem Innenschaft in Eingriff stehen können, um den Innenschaft in seiner Relativdrehstellung bezüglich des Außenschafts zu sichern und sein ungewolltes Verdrehen zu verhindern. Eine vollständige Drehung des Innenschafts relativ zum Außenschaft um 360° wird hierbei durch mehrere unmittelbar aneinander anschließende Teildrehungen des Innenschafts bewirkt, die jeweils durch ein Mitnehmerelement vermittelt werden. Es ist insbesondere möglich, dass der Innenschaft des medizinischen Instruments in beide Drehrichtungen um die Längsachse drehbar ist.

Die Ausgestaltung des proximalen Endbereichs des Außenschafts mit umfänglich begrenzten Öffnungen, durch die sich die Mitnehmerelemente hindurch erstrecken, bewirkt, dass der proximale Endbereich des Außenschafts einteilig ausgebildet ist und sich über die Drehübertragungsmechnik des medizinischen Instruments hinaus erstreckt, wodurch der proximale Endbereich des Außenschafts mit einem bereits vorhandenen Handgriff verbindbar ist, ohne diesen umzugestalten. Hierdurch weist das medizinische Instrument vorteilhafterweise geringe Fertigungskosten auf.

Da die Mitnehmerelemente des medizinischen Instruments distalseitig des proximalen Endes des Außenschafts angeordnet sind, ist es ferner möglich, das Stellglied auch distalseitig des Handgriffs anzuordnen, so dass das medizinische Instrument vorteilhafterweise durch einen Benutzer einhändig bedienbar ist.

In einer bevorzugten Ausgestaltung der Erfindung weist der Außenschaft benachbart zu den Öffnungen des Außenschafts Steuerkurven für die Mitnehmerelemente auf, die zum abwechselnden Außer-Eingriff- und In-Eingriff-Bringen der Mitnehmerelemente mit dem Innenschaft dienen.

Die an dem Außenschaft axial benachbart zu den Öffnungen angeordneten Steuerkurven bewirken eine lösbare Verbindung der Mitnehmerelemente mit dem Innenschaft, so dass nur abwechselnd eines der Mitnehmerelemente zur Übertragung der Drehbewegung mit dem Innenschaft in Eingriff steht. Dies ermöglicht vorteilhafterweise eine konstruktiv einfache und kompakte Bauweise des medizinischen Instruments, da der Außenschaft nicht nur dem Schutz des Innenschafts vor einer Beschädigung, sondern auch gleichzeitig zum Außer-Eingriff- und In-Eingriff-Bringen der Mitnehmerelemente dient. Ferner stellen die Steuerkurven vorteilhafterweise eine umfängliche Führung der Mitnehmerelemente bereit. Die Steuerkurven des Außenschafts können bspw. als Nocken ausgebildet sein, deren Außenflächen im Umfangsrichtung des Schafts gesehen verschieden weit radial vom Innenschaft beabstandet sind.

In einer weiteren bevorzugten Ausgestaltung der Erfindung sind die Steuerkurven vollumfänglich ausgebildet und in ihren nicht an den Öffnungen anliegenden Abschnitten derart radial vom Innenschaft beabstandet, dass die Mitnehmerelemente außer Eingriff mit dem Innenschaft gebracht werden.

Diese Maßnahme bewirkt, dass die Außenflächen der Steuerkurven im Bereich ihrer nicht an den Öffnungen anliegenden Abschnitten derart radial vom Innenschaft beabstandet sind, dass die Mitnehmerelemente, die durch die Steuerkurven geführt werden, in diesen Abschnitten von dem Innenschaft entfernt werden. Hierdurch werden die Mitnehmerelemente außer Eingriff mit dem Innenschaft gebracht, so dass jedes Mitnehmerelement nur eine Teildrehung des Innenschafts relativ zum Außenschaft bewirkt und eine Drehung des Innenschafts um 360° durch die einzelnen Teildrehungen der jeweiligen Mitnehmerelemente ermöglicht wird. Die vollumfängliche Ausgestaltung der Steuerkurven ermöglicht vorteilhafterweise zusätzlich eine vollumfängliche Führung der Mitnehmerelemente in Umfangsrichtung des Schafts, wodurch die Anordnung der Mitnehmerelemente während der Drehung umfänglich gesehen stabil ist.

In einer weiteren bevorzugten Ausgestaltung der Erfindung sind die Mitnehmerelemente umfänglich versetzt angeordnet.

Diese Maßnahme hat den Vorteil, dass die Fertigung des Außenschafts des medizinischen Instruments besonders einfach ist, da die axial versetzten Öffnungen des Außenschafts an einer Umfangsstelle des Außenschafts zueinander axial benachbart angeordnet sind. Zusätzlich wird vorteilhafterweise das Gewicht der Mitnehmerelemente im Umfangsrichtung des Schafts gesehen gleichmäßig verteilt, wodurch das medizinische Instrument einfach von dem Benutzer gehalten werden kann.

In einer weiteren bevorzugten Ausgestaltung der Erfindung sind die Mitnehmerelemente jeweils zweiteilig ausgebildet, wobei ein erstes Mitnehmerelementteil und zweites Mitnehmerelementteil der Mitnehmerelemente mit Federkraft beaufschlagbar sind, wobei das erste Mitnehmerelementteil und zweite Mitnehmerelementteil in radialer Richtung relativ zueinander beweglich sind.

Diese Maßnahme bewirkt, dass die Mitnehmerelemente in radialer Richtung längenverstellbar sind, so dass sich die Ausdehnung der Mitnehmerelemente vorteilhafterweise an den Verlauf der Steuerkurven anpasst und sich wahlweise während ihres Außer-Eingriff- und In-Eingriff-Bringens verkürzen und verlängern kann. Bei einer Ausgestaltung des medizinischen Instruments, bei dem Mitnehmerelementenden in dem Stellglied aufgenommen sind, ermöglicht die längenverstellbare Ausgestaltung der Mitnehmerelemente ferner, dass sich diejenigen Mitnehmerelemente, die gerade nicht mit dem Innenschaft in Eingriff stehen, verkürzen und folglich nicht aus dem Stellglied vorspringen. Hierdurch wird vorteilhafterweise eine besonders sichere Bedienung des medizinischen Instruments gewährleistet.

Alternativ und ebenso vorteilhaft können die Mitnehmerelemente auch einteilig ausgebildet sein, wobei die Mitnehmerelemente über eine Zwangssteuerung mit dem Innenschaft in und außer Eingriff gebracht werden können. Eine solche Zwangssteuerung kann bspw. durch Führungen realisiert sein, die von der Seite in die Mitnehmerelemente greifend diese in Richtung Mittelachse bzw. von dieser wegbewegen können.

In einer weiteren bevorzugten Ausgestaltung der Erfindung erstrecken sich die Mitnehmerelemente bezüglich des Schafts in radialer Richtung.

Diese Maßnahme hat den Vorteil, dass das medizinische Instrument im Bereich der Drehübertragungsmechanik besonders stabil und kompakt ausgebildet ist, da der Innenschaft, der Außenschaft und das Stellglied besonders nah beabstandet angeordnet sein können. Ferner bewirkt die radiale Anordnung der Mitnehmerelemente bezüglich des Schafts eine besonders große Hebelwirkung zwischen dem Stellglied und dem Innenschaft und somit einen besonders effektiven Drehübertrag auf den Innenschaft, wodurch vorteilhafterweise nur eine geringe Kraft zum Drehen des Innenschafts auf das Stellglied ausgeübt werden muss. Dies ist insbesondere bei einer Ausgestaltung des Stellglieds als manuell betätigbares Handrad vorteilhaft, da der Handgriff des Instrument durch den Benutzer mit einer Hand gehalten und zugleich das Stellglied ohne große Kraftanstrengung gedreht werden kann.

In einer weiteren bevorzugten Ausgestaltung der Erfindung weist der Innenschaft zumindest eine Aussparung zur Aufnahme der Mitnehmerelemente auf.

Das Vorsehen einer Aussparung im Innenschaft ermöglicht vorteilhafterweise das In-Eingriff-Bringen der Mitnehmerelemente mit dem Innenschaft auf konstruktiv sehr einfache Weise, da ein Mitnehmerelementende direkt in die Aussparung eingreifen kann. Ferner ermöglicht das direkte Eingreifen der Mitnehmerelemente in die Aussparung des Innenschafts eine kostengünstige Fertigung des medizinsichen Instruments, da zur Erzeugung der drehfesten Verbindung des Stellglieds und des Innenschafts keine weiteren Bauteile vorhanden sein müssen.

Im Zusammenhang mit der umfänglich versetzten Anordnung der Mitnehmerelemente weist der Innenschaft vorzugsweise mehrere entsprechend umfänglich versetzte Aussparungen auf, in die jeweils ein oder abwechselnd mehrere Mitnehmerelemente eingreifen können.

In einer weiteren bevorzugten Ausgestaltung der Erfindung ist die Aussparung als in die Längsrichtung verlaufende Nut ausgebildet.

Diese Maßnahme ermöglicht vorteilhafterweise eine besonders einfache und kostengünstige Fertigung des Innenschafts, indem bspw. die Nut oberflächlich aus einer Innenschaftwandung herausgefräst wird. Die axial verlaufende Ausgestaltung der Nut ermöglicht ferner vorteilhafterweise ein sicheres Halten der Mitnehmerelemente, da die in die Nut eingreifenden Mitnehmerelementenden nicht in Umfangsrichtung des Schafts gesehen verrutschen können. Im Bezug auf eine axial benachbarte Anordnung der axial versetzten Mitnehmerelemente ermöglicht die Ausgestaltung der Aussparung als Nut das abwechselnde Eingreifen der Mitnehmerelemente in eine einzige Aussparung, wodurch das medizinische Instrument vorteilhafterweise konstruktiv sehr einfach ausgebildet ist und zugleich kostengünstig gefertigt werden kann.

Die Aussparung des Innenschafts kann auch als durch den Innenschaft hindurch reichendes Loch oder als Vertiefung beliebiger Form ausgestaltet sein, dessen bzw. deren Querschnitt an ein in die Aussparung eingreifendes Mitnehmerelementende angepasst ist.

In einer weiteren bevorzugten Ausgestaltung der Erfindung sind die Öffnungen des Außenschafts als Schlitze ausgebildet.

Eine Ausgestaltung der Öffnungen als bspw. langlochförmige Schlitze stellt eine umfängliche Führung der durch die Schlitze hindurchreichenden Mitnehmerelemente bereit, wodurch die Mitnehmerelemente vorteilhafterweise axial lagestabil gehalten werden und ihr axiales Verkippen verhindert wird. Diese Maßnahme ermöglicht ferner eine besonders einfache Fertigung des Außenschafts, da das Einbringen von umfänglich begrenzten, länglichen Schlitzen in den Außenschaft besonders einfach durchgeführt werden kann. Die umfängliche Ausdehnung der Schlitze wird dabei durch die zur Stabilität des Außenschafts notwendigen umfänglichen Außenschaftteilbereiche bestimmt, die sich jeweils umfänglich gesehen an die Schlitze anschließen.

In einer weiteren bevorzugten Ausgestaltung der Erfindung weist das medizinische Instrument genau zwei Mitnehmerelemente auf, wobei die Öffnungen des Außenschafts etwa halbumfänglich ausgebildet sind.

Die Ausgestaltung des medizinischen Instruments mit nur zwei Mitnehmerelementen stellt die einfachste Möglichkeit dar, eine Drehübertragungsmechanik zum Drehen des Innenschafts relativ zum Außenschaft zu schaffen, wodurch das medizinische Instrument vorteilhafterweise konstruktiv sehr einfach mit wenigen Bauteilen gefertigt ist und die Fertigungskosten des medizinischen Instruments ferner im Vergleich zu einer Ausgestaltung der Drehübertragungsmechanik mit mehr als zwei Mitnehmerelementen geringer sind. Eine Relativdrehung des Innenschafts bezüglich des Außenschafts um einen Winkel von 360° wird hierbei durch zwei Teildrehungen des Innenschafts um jeweils einen Winkel von 180° bewirkt, die jeweils durch das In-Eingriff-Stehen eines der beiden Mitnehmerelemente mit dem Innenschaft erzielt werden.

In einer weiteren bevorzugten Ausgestaltung der Erfindung sind die zwei Mitnehmerelemente umfänglich um etwa 180° versetzt angeordnet.

Diese Maßnahme hat den Vorteil, dass die auf dem Innenschaft wirkenden Drehkräfte an zwei in Umfangsrichtung des Schafts gesehen gegenüberliegenden Stellen des Innenschafts angreifen, wodurch die Drehbewegung des Innenschafts vorteilhafterweise besonders gleichmäßig ist. Bei einer umfänglich um etwa 180° versetzten Anordnung der zwei Mitnehmerelemente weist das medizinische Instrument vorzugsweise genau zwei Aussparungen auf, in die jeweils abwechselnd ein zum Innenschaft weisendes Ende eines Mitnehmerelements eingreift.

In einer weiteren bevorzugten Ausgestaltung der Erfindung sind die zwei Mitnehmerelemente axial benachbart angeordnet.

Bei einer axial benachbarten Anordnung der zwei Mitnehmerelemente sind die beiden Mitnehmerelemente etwa an der gleichen Umfangsstelle des Schafts angeordnet. Diese Maßnahme hat den Vorteil, dass das medizinische Instrument besonders kostengünstig gefertigt werden kann, da der Innenschaft nur eine Aussparung für beide Mitnehmerelemente aufweisen muss. Die Öffnungen des Außenschafts sind hierbei um 180° umfänglich versetzt angeordnet und die Steuerkurven des Außenschafts sind ferner gegenläufig ausgebildet und ebenfalls um 180° versetzt angeordnet.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit der beiliegenden Zeichnung näher beschrieben und erläutert. Es zeigen:
- Fig. 1: eine perspektivische Ansicht eines erfindungsgemäßen medizinischen Instruments;
- Fig. 2: eine Querschnittsansicht eines Handgriffs des medizinischen Instru- ments in Fig. 1;
- Fig. 3: eine perspektivische Ansicht eines proximalen Endes eines Innen- schafts des medizinischen Instruments in Fig. 1;
- Fig. 4: eine perspektivische Ansicht eines proximalen Endes eines Außen- schafts des medizinischen Instruments in Fig. 1;
- Fig. 5: eine Querschnittsansicht eines Mitnehmerelements;
- Fig. 6: eine Querschnittsansicht einer Steuerkurve;
- Fig. 7: eine vergrößerte Querschnittsansicht des Handgriffs in Fig. 2;
- Fig. 8A-C: perspektivische Ansichten des Handgriffs in Fig. 2 in verschiedenen Drehstellungen; und
- Fig. 9A-C: Querschnittsansichten des Handgriffs in Fig. 8A-C in den verschiede- nen Drehstellungen.

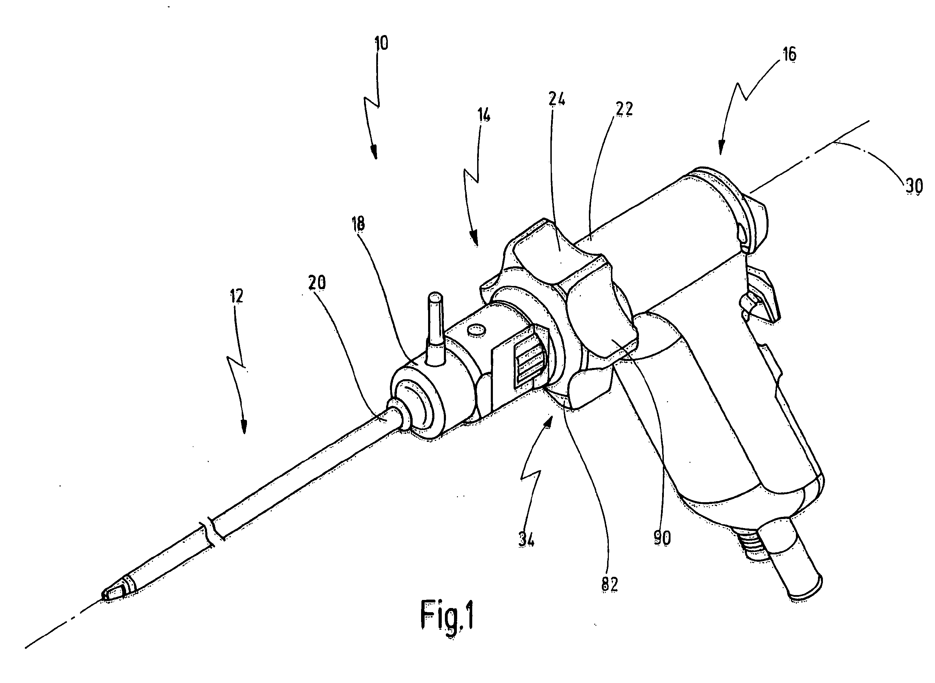

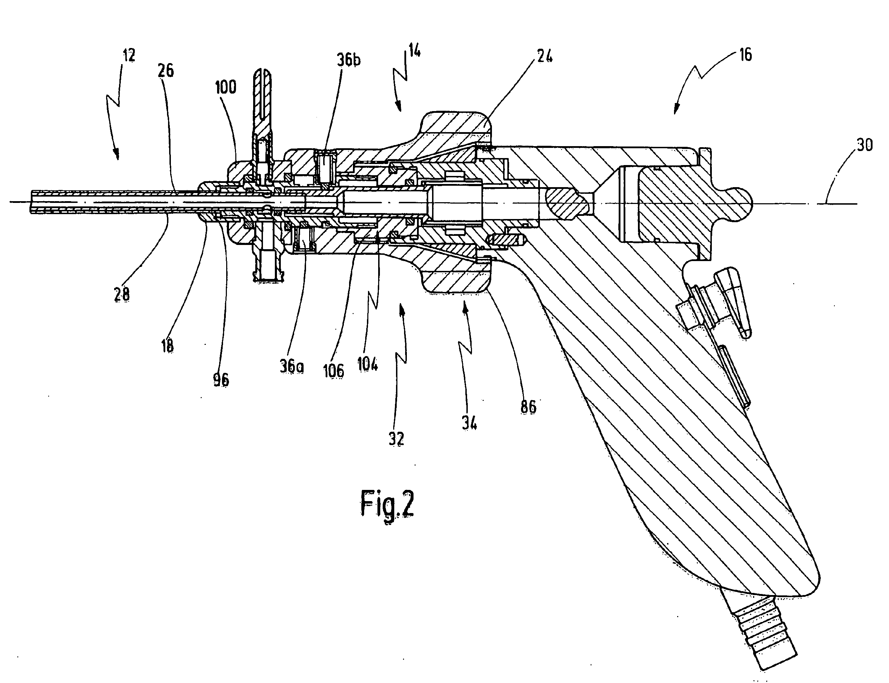

In Fig. 1 ist ein mit dem allgemeinen Bezugszeichen 10 versehenes medizinisches Instrument dargestellt. Weitere Einzelheiten des medizinischen Instruments 10 sind in den Fig. 2 - 9C dargestellt.

Das medizinische Instrument 10 wird bspw. im Bereich der minimal-invasiven Chirurgie während eines laparoskopischen Eingriffs zum Veröden von geschnittenem Gewebe eines Patienten oder zum Abtragen von krankhaftem Gewebe des Patienten verwendet.

Das medizinische Instrument 10 weist einen Schaft 12, ein Kupplungselement 14 und einen Handgriff 16 auf. Ein distaler Endbereich 18 des Kupplungselements 14 ist mit einem proximalen Endbereich 20 des Schafts 12 und ein distaler Endbereich 22 des Handgriffs 16 ist mit einem proximalen Endbereich 24 des Kupplungselements 14 verbunden.

Wie in Fig. 2 gezeigt, ist der Schaft 12 zweiteilig ausgebildet, und er weist einen jeweils rohrförmigen Innenschaft 26 und Außenschaft 28 auf. Der Innenschaft 26 ist in dem Außenschaft 28 aufgenommen und relativ zum Außenschaft 28 um eine Längsachse 30 des medizinischen Instruments 10 in beide Drehrichtungen um jeweils 360° drehbar ausgebildet. In dem gezeigten Ausführungsbeispiel entspricht die Längsachse 30 des medizinischen Instruments 10 einer Längsachse des Schafts 12. Der Außenschaft 28 ist drehfest mit dem Kupplungselement 14 verbunden. Es ist ebenfalls möglich, dass der Außenschaft 28 um die Längsachse 30 beispielsweise schwergängig drehbar ist und der Innenschaft 26 relativ zum drehbaren Außenschaft 28 drehbar ist.

Zum Erzeugen einer Relativdrehbewegung des Innenschafts 26 und des Außenschafts 28 weist das Kupplungselement 14 eine Drehübertragungsmechanik 32 mit einem Stellglied 34 und zumindest zwei Mitnehmerelementen 36, hier genau zwei Mitnehmerelementen 36a, b, auf, die zur Übertragung der Relativdrehbewegung jeweils abwechselnd mit dem Innenschaft 26 in Eingriff stehen.

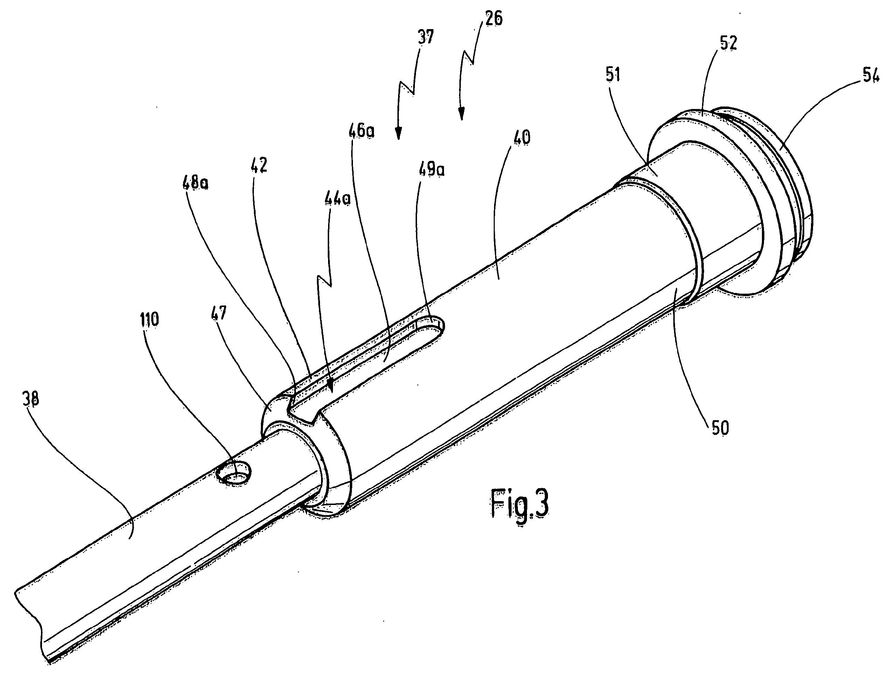

Bezug nehmend auf Fig. 3 - 7 werden im Folgenden der Innenschaft 26, der Außenschaft 28, die Mitnehmerelemente 36a, b und das Stellglied 34 genauer beschrieben.

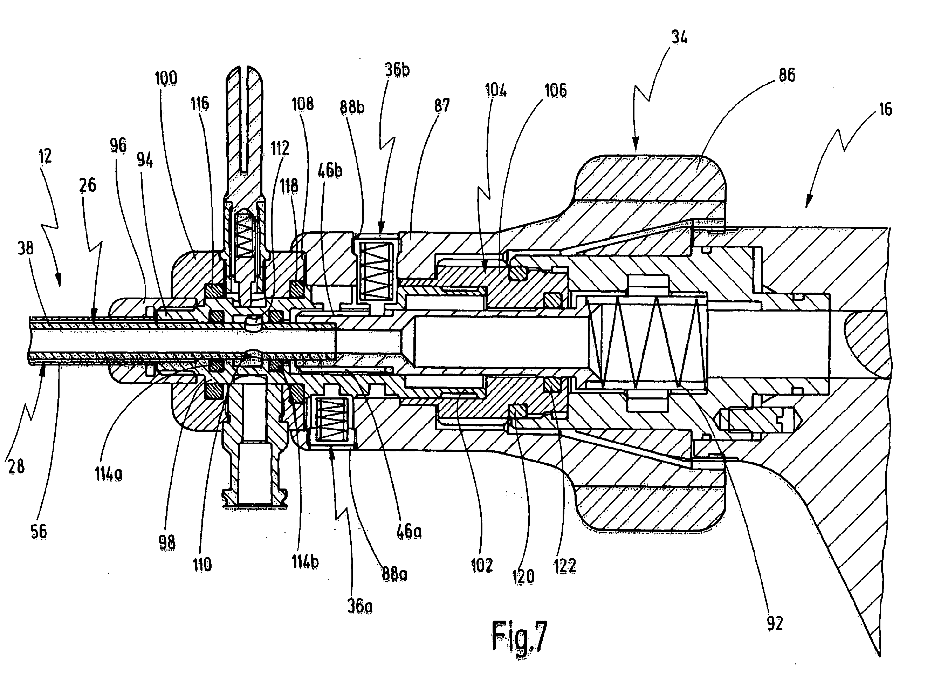

Wie in Fig. 3 gezeigt, ist ein proximaler Endbereich 37 des Innenschafts 26 zweiteilig ausgebildet und weist ein Innenrohr 38 und eine rohrförmige Innenrohraufnahme 40 auf, die bspw. durch Verschweißen, ggf. auch Verlöten proximalseitig bzw. distalseitig drehfest miteinander verbunden sind. Das Innenrohr 38 und die Innenrohraufnahme 40 können auch einstückig ausgebildet sein. Der Außendurchmesser des Innenrohrs 38 ist um etwa die Hälfte kleiner als der Außendurchmesser der Innenrohraufnahme 40, so dass sich der Innenschaft 26 im Bereich des Übergangs zwischen dem Innenrohr 38 und der Innenrohraufnahme 40 stufenförmig verbreitert. In einem distalen Endbereich 42 der Innenrohraufnahme 40 sind zwei umfänglich um etwa 180° zueinander versetzte Aussparungen 44a, b in Form zweier in die Längsrichtung 30 des medizinischen Instruments 10 verlaufender, axial begrenzter Nuten 46a, b vorgesehen. Ein distales Ende 47 des distalen Endbereichs 42 der Innenrohraufnahme 40 ist vollumfänglich nach distal hin abgeschrägt, so dass sich das distale Ende 47 der Innenrohraufnahme 40 nach distal hin verjüngt und ein distales Ende 48a, b der Nuten 46a, b offen ist bzw. keine Wandung aufweist. Ein proximales Ende 49a, b der Nuten 46a, b ist halbkreisförmig ausgestaltet. Die Nuten 46a, b sind bspw. aus dem distalen Endbereich 42 der Innenrohraufnahme 40 herausgefräst. Hierbei ist eine Wandstärke des distalen Endbereichs 42 der Innenrohraufnahme 40 größer als die Wandstärke des Innenrohrs 38 und eines restlichen Abschnitts der Innenrohraufnahme 40 ausgebildet, da der Innendurchmesser des Innenrohrs 38 etwa dem Innendurchmesser der Innenrohraufnahme 40 im Bereich der Nuten 46a, b entspricht (vgl. Fig. 7). Ein Inneres der Innenrohraufnahme 40 verbreitert sich ferner trichterförmig nach proximal hin, so dass der Innendurchmesser der Innenrohraufnahme 40 stufenförmig zunimmt und die Innenrohraufnahme 40 und das Innenrohr 38 außer im Bereich der beiden Nuten 46a, b eine annähernd gleiche Wandstärke besitzen. Die Aussparungen 44a, b können auch als durch die Innenrohraufnahme 40 hindurchreichende Löcher oder als Vertiefungen beliebiger Form ausgebildet sein.

An einem proximalen Endbereich 50 der Innenrohraufnahme 40 ist ein Bund 51, ein Kragen 52 sowie ein weiterer Bund 54 angeordnet. Der Außendurchmesser des Bundes 54 bzw. der Außendurchmesser des Bundes 51 ist größer als der Außendurchmesser des Bundes 51 bzw. der Außendurchmesser der Innenrohraufnahme 40. Der Außendurchmesser des Kragens 52 ist größer als der Außendurchmesser des Bundes 54.

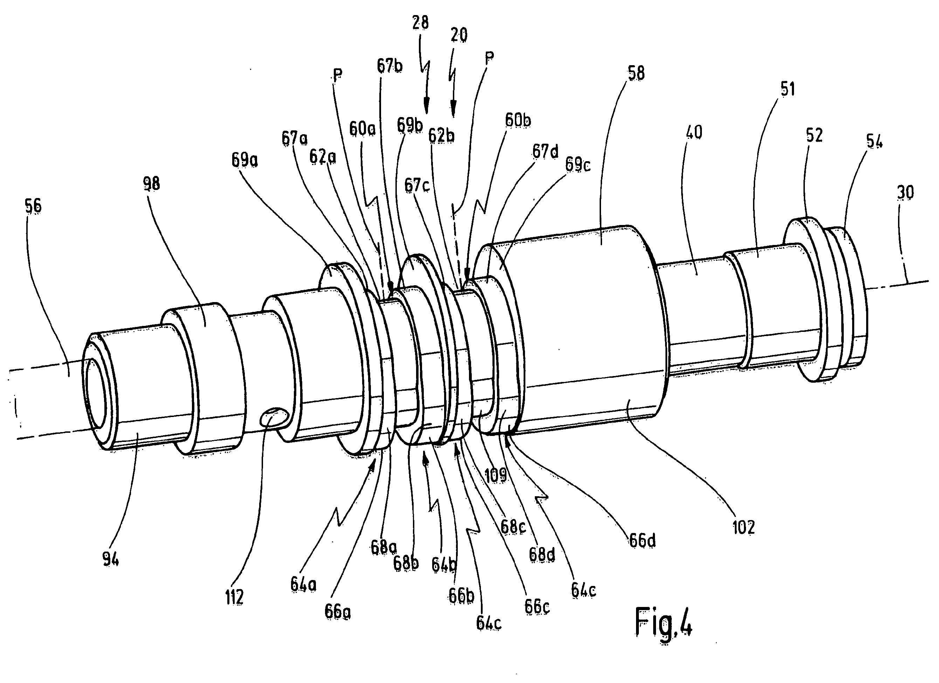

Der Außenschaft 28 ist ebenfalls zweiteilig ausgebildet und weist ein Außenrohr 56 und eine Außenrohraufnahme 58 auf, die proximalseitig bzw. distalseitig drehfest miteinander verbunden sind (vgl. Fig. 4). Das Außenrohr 56 und die Außenrohraufnahme 58 können ebenfalls wie das Innenrohr 38 und die Innenrohraufnahme 40 einstückig ausgebildet sein. Die Außenrohraufnahme 58 weist etwa mittig zwei Öffnungen 60a, b in Form zweier etwa halbumfänglicher, langlochförmiger Schlitze 62a, b auf, die axial benachbart an etwa der gleichen Umfangsstelle der Außenrohraufnahme 58 angeordnet sind. Die Schlitze 62a, b können auch in Umfangsrichtung des Schafts 12 gesehen einen Öffnungsbereich von etwa 220° oder auch etwa 270° aufweisen. Der maximale Öffnungsbereich der Schlitze 62a, b wird hierbei durch einen minimal zur Stabilität der Außenrohraufnahe 58 notwendigen Rohrbereichsteilumfang bestimmt, der umfänglich benachbart zu den Schlitzen 62a, b angeordnet ist. Die Wandstärke der Außenrohraufnahme 58 ist in Bereich der beiden Schlitze 62a, b geringer als die Wandstärke der Außenrohraufnahme 58 in ihrem zu den Schlitzen 62a, b umfänglich versetzten Bereich. Der distalseitige Innendurchmesser der Außenrohraufnahme 58 entspricht etwa dem Außendurchmesser des Innenrohrs 38. Ein Inneres der Außenrohraufnahme 58 verbreitert sich ferner stufenförmig nach proximal hin, so dass der Innendurchmesser der Außenrohraufnahme 58 im Bereich der Schlitze 62a, b etwa dem Außendurchmesser der Innenrohraufnahme 40 entspricht.

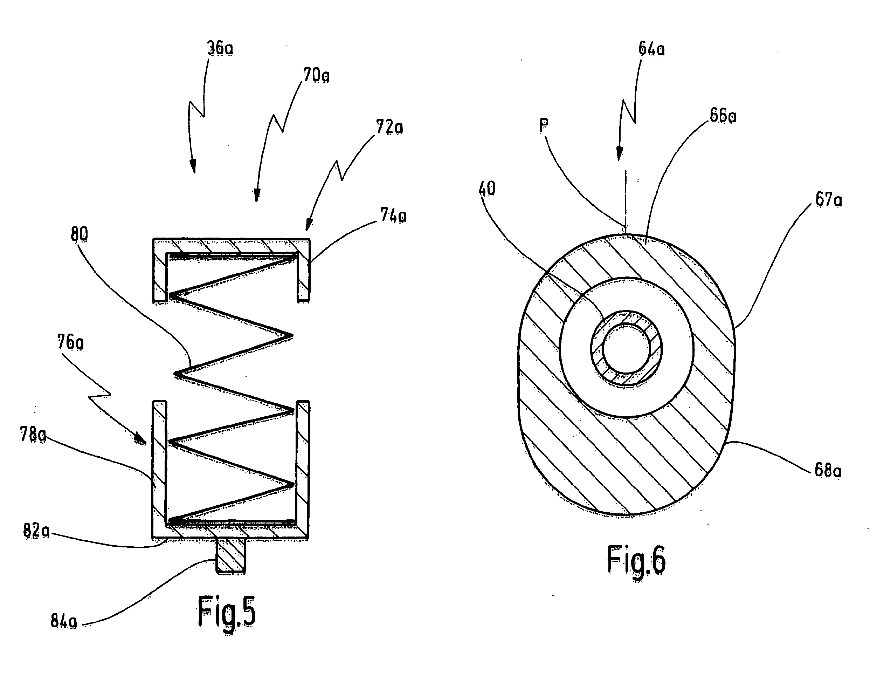

Die Außenrohraufnahme 58 weist ferner beidseitig der zwei Schlitze 62a, b jeweils zwei Steuerkurven 64a-d auf, die die Außenrohraufnahme 58 vollumfänglich umgreifen und zueinander axial benachbart angeordnet sind. Wie in Fig. 5 gezeigt, sind die Steuerkurven 64a-d als zueinander symmetrisch verlaufende Nocken 66a-d ausgebildet. Im Bereich der beiden Schlitze 62a, b entsprechen die Nocken 66a-d einer Außenfläche der rohrförmigen Außenrohraufnahme 58. Der radiale Abstand vollumfänglicher Außenflächen 67a-d der Nocken 66a-d von der Innenrohraufnahme 40 variiert in Umfangsrichtung des Schafts 12, um ein In-Eingriff-Bringen und Außer-Eingriff-Bringen der Mitnehmerelemente 36a, b mit den Nuten 46a, b zu ermöglichen. Im Bereich der Schlitze 62a, b ist der radiale Abstand der Außenflächen 67a-b von der Innenrohraufnahme 40 annährend konstant, während der radiale Abstand von nicht an den Schlitzen 62a, b anliegenden Abschnitten 68a-d der Außenflächen 67a-d der Nocken 66a-d von der Innenrohraufnahme 40 in diesem Bereich mit dem Abstand von den Schlitzen zunimmt. Die Abschnitte 68a-d der Außenflächen 67a-d der Nocken 66a-d weisen in einem um etwa 180° zu einem Schlitzmittelpunkt P umfänglich versetzten Bereich den größten radialen Abstand von der Innenrohraufnahme 40 auf.

Die Außenrohraufnahme 58 weist ferner drei ringförmige Führungselemente 69a-c für die Mitnehmerelemente 36a, b auf, deren annähernd konstanter Außendurchmesser größer als der Außendurchmesser der Außenrohraufnahme 58 ist. Das erste Führungselement 69a ist distalseitig direkt benachbart zur Nocke 66a, das zweite Führungselement 69b ist zwischen den Nocken 66b, c und das dritte Führungselement 69c ist proximalseitig direkt benachbart zur Nocke 66d angeordnet. Es ist ebenfalls möglich, dass die Nocken 66b, c direkt benachbart angeordnet sind und die Außenrohraufnahme 58 nur die zwei Führungselemente 69a, c aufweist.

Die Mitnehmerelemente 36a, b sind als zweiteilige, zylinderförmige Stifte 70a, b ausgebildet, die sich in radialer Richtung des Schafts 12 erstrecken (vgl. Fig. 5). Ein erstes Mitnehmerelementteil 72a, b ist als Kappe 74a, b und ein zweites Mitnehmerelementteil 76a, b ist als hohlzylinderförmiges Unterteil 78a, b ausgebildet. Zwischen der Kappe 74a, b und dem Unterteil 78a, b ist eine Feder 80a, b aufgenommen, so dass die Stifte 70a, b in radialer Richtung des Schafts 12 längenverstellbar sind. Ferner weist jedes Unterteil 78a, b an seiner Stirnseite 82a, b, die in einem zusammengebauten Zustand des medizinischen Instruments 10 zum Innenschaft 26 weist, einen länglichen zylinderförmigen Fortsatz 84a, b auf.

Wie in Fig. 7 gezeigt, ist das Stellglied 34 als Handrad 86 ausgebildet, in dem der proximale Endbereich 20 des Schafts 12 aufgenommen ist. In einem distalen Endbereich 87 des Handrads 86 sind zwei in der Längsrichtung 30 und umfänglich um etwa 180° zueinander versetzte Durchbohrungen 88a, b vorhanden. Das Handrad 86 weist ferner flügelartige Mulden 90 auf, an denen ein Benutzer angreifen kann.

Das Innenrohr 38, die Innenrohraufnahme 40, das Außenrohr 56, die Außenrohraufnahme 58, die Mitnehmerelemente 36a, b, die Steuerkurven 64a-d und das Stellglied 34 sind vorzugsweise aus beispielsweise rostfreiem Metall gefertigt.

Fig. 7 zeigt das medizinische Instrument 10 in seinem zusammengebauten Zustand, bei dem der Innenschaft 26 in dem Außenschaft 28 aufgenommen ist. Hierbei liegt das distale Ende 47 der Innenrohraufnahme 40 an dem stufenförmig verbreiterten Bereich des Inneren der Außenrohraufnahme 58 an. Eine Feder 92 ist zwischen dem proximalen Endbereich 50 der Innenrohraufnahme 40 und dem Handgriff 16 aufgenommen. Die Feder 92 drückt proximalseitig auf den Kragen 52 der Innenrohraufnahme 40, so dass der Innenschaft 26 axial lagestabil bezüglich des Außenschafts 28 angeordnet ist.

Ein distaler Endbereich 94 der Außenrohraufnahme 58 ist beispielsweise durch Verschrauben außenseitig drehfest mit einem Verbindungsteil 96 des Kupplungselements 14 verbunden. Das Verbindungsteil 96 und ein an den distalen Endbereich 94 der Außenrohraufnahme 58 angrenzender Bereich 98 der Außenrohraufnahme 58 sind ferner beispielsweise durch Verschrauben drehfest mit einem Anschlussteil 100 des Kupplungselements 14 verbunden. Das Anschlussteil 100 kann Anschlüsse für Saug- oder Spülleitungen oder auch einen Hochfrequenzstromanschluss für eine Elektrode aufweisen, die an einem distalen Ende des Schafts 12 angeordnet sein kann. Ein proximaler Endbereich 102 der Außenrohraufnahme 58 ist ferner über ein Verbindungselement 104 innenseitig drehfest mit dem Handgriff 16 verbunden. Das Verbindungselement 104 kann beispielsweise eine Mutter 106 sein, die mit dem Handgriff 16 verschraubbar ist.

Die Innenrohraufnahme 40 ist über die Mitnehmerelemente 36a, b drehfest mit dem Handrad 86 verbunden, so dass eine Drehung des Handrads 86 direkt auf die Innenrohraufnahme 40 übertragen wird und sich das drehfest mit der Innenrohraufnahme 40 verbundene Innenrohr 38 relativ zum Außenschaft 28 mitdreht. Das Handrad 86 ist dabei innenseitig derart radial von dem Handgriff 16, dem Verbindungselement 104 und der Außenrohraufnahme 58 beabstandet, dass keine Reibungskräfte an diesen Bauteilen auftreten, die die Drehung des Handrads 86 beeinträchtigen können. In dem distalen Endbereich 87 des Handrads 86 ist ein proximaler Endbereich 108 des Anschlussteils 100 vollumfänglich aufgenommen, zu dem das Handrad 86 frei drehbar ist.

Die Mitnehmerelemente 36a, b sind in den Durchbohrungen 88a, b des Handrads 86 aufgenommen. Die Kappen 74a, b der Stifte 70a, b sind dabei radial fest angeordnet, wodurch die Unterteile 78a, b in radialer Richtung des Schafts 12 relativ zur Kappe 74a, b bewegbar und die Stifte 70a, b längenverstellbar ausgebildet sind. Die Fortsätze 84a, b der Unterteile 78a, b reichen durch die Schlitze 62a, b der Außenrohraufnahme 58 hindurch und greifen abwechselnd in die Nuten 46a, b ein. Die Stirnseiten 82a, b der Unterteile 78a, b liegen dabei an den Außenflächen 67a-d der Nocken 66a-d an und werden von diesen umfänglich geführt. Der radiale Abstand der Außenflächen 67a-d der Nocken 66a-d von der Innenrohraufnahme 40 im Bereich der Schlitze 62a, b ist hierbei derart bemessen, dass die Fortsätze 84a, b der Stifte 70a, b vollständig in Nuten 46a, b eingreifen und fast einen Nutboden berühren. Der radiale Abstand der Außenflächen 67a-d der Nocken 66a-d von der Innenrohraufnahme 40 im Bereich der nicht an den Schlitzen 62a, b anliegenden Abschnitten 68a-d ist hingegen derart bemessen, dass die Fortsätze 84a, b der Stifte 70a, b aus den Nuten 46a, b heraus gehoben werden und an einer Außenfläche 109 der Außenrohraufnahme 58 anliegen. Das erste Mitnehmerelement 36a wird seitlich durch das erste und zweite Führungselement 69a, b und das zweite Mitnehmerelement 36b wird durch das zweite und dritte Führungselement 69b, c geführt, so dass eine axiale Verkippung der beiden Mitnehmerelemente 36a, b verhindert wird.

Die Mitnehmerelemente 36a, 36b können anstelle zweiteilig mit Federbeaufschlagung auch einteilig ausgebildet sein, wobei die Mitnehmerelemente 36a, 36b über eine Zwangssteuerung mit dem Innenschaft in und außer Eingriff gebracht werden können. Eine solche Zwangssteuerung kann bspw. durch Führungen realisiert sein, die von der Seite in die Mitnehmerelemente 36a, 36b greifend diese in Richtung Achse bzw. von dieser wegbewegen können.

Zwischen der Außenrohraufnahme 58 und dem Innenrohr 38 sind beidseitig einer durch das Innenrohr 38 hindurchgehenden Innenrohröffnung 110 und einer im zusammengebauten Zustand des medizinischen Instruments 10 radial benachbart angeordneten, durch die Außenrohraufnahme 58 hindurchgehenden Außenrohraufnahmeöffnung 112 zwei vollumfängliche Dichtungen 114a, b angeordnet, die die Dichtigkeit zwischen den angrenzenden Bauteilen des medizinischen Instruments 10 erhöhen. Ferner weist das Kupplungselement 14 zwischen dem Anschlussteil 100 und der Außenrohraufnahme 58 eine weitere vollumfängliche Dichtung 116 auf, die in radialer Richtung des Schafts 12 benachbart zur Dichtung 114a angeordnet ist. Eine weitere vollumfängliche Dichtung 118 ist zwischen der Außenrohraufnahme 58 distalseitig des ersten Führungselements 69a, dem Anschlussteil 100 und dem distalen Endbereich 87 des Handrads 86 angeordnet. Im Bereich des proximalen Endbereichs 94 der Außenrohraufnahme 58 sind ferner zwei weitere vollumfängliche Dichtungen 120, 122 angeordnet, die die Außenrohraufnahme 58 bzw. das mit ihm verbundene Verbindungselement 104 gegenüber dem Handgriff 16 und den Bund 51 der Innenrohraufnahme 40 gegenüber der Außenrohraufnahme 58 abdichten. Die Dichtungen 114a, b, 116 - 122 sind aus Gummi gefertigt.

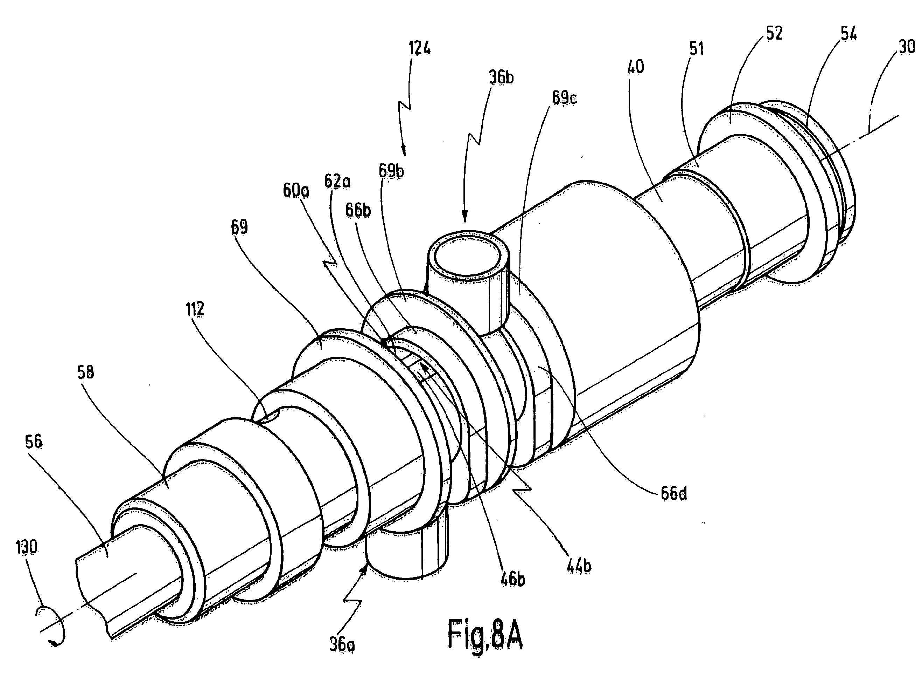

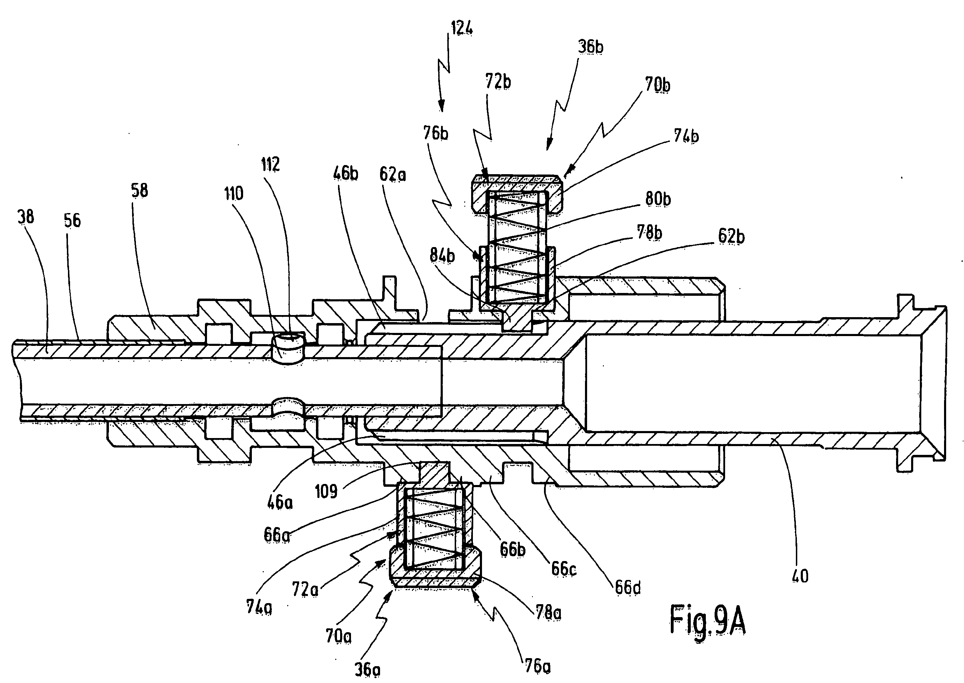

Fig. 8A-C und Fig. 9A-C zeigen perspektivische Ansichten und Querschnittsansichten des Handgriffs 16 bzw. Drehmechanismus in drei verschiedenen Relativdrehstellungen 124 - 128 des Innenschafts 26 und des Außenschafts 28. Die zugehörigen Relativdrehwinkel des Innenschafts 26 und Außenschafts 28 betragen etwa 0°, 90° und 180°.

In der in den Fig. 8A und 9A dargestellten ersten Drehstellung 124 greift der Fortsatz 84b des Stifts 70b durch den Schlitz 62b hindurch vollständig in die Nut 46b der Innenrohraufnahme 40 ein, während der Fortsatz 84a des Stifts 70a aufgrund der radialen Beabstandung der Nocken 66a, b an der Außenfläche 109 der Außenrohraufnahme 58 aufsitzt und keine Verbindung zur Innenrohraufnahme 40 hat. Das Unterteil 78a des Stifts 70a ist zur radial fest angeordneten Kappe 74a hingedrückt, so dass der längenverstellbare Stift 70a seine minimale Länge besitzt. Der Stift 70b hingegen weist seine maximale Länge auf, so dass sein Unterteil 78b von der Kappe 74b maximal beabstandet ist.

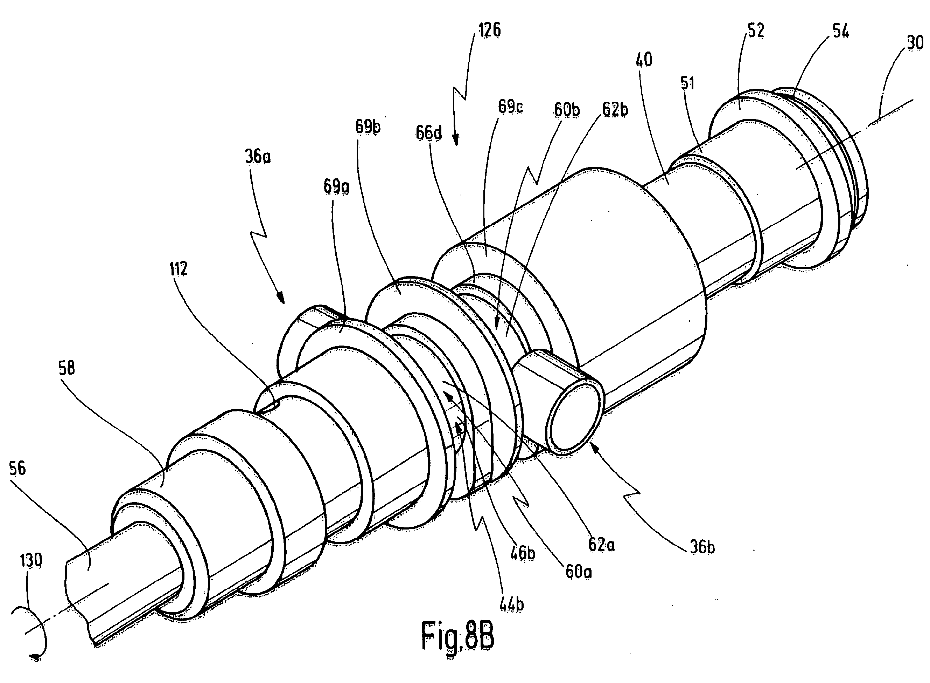

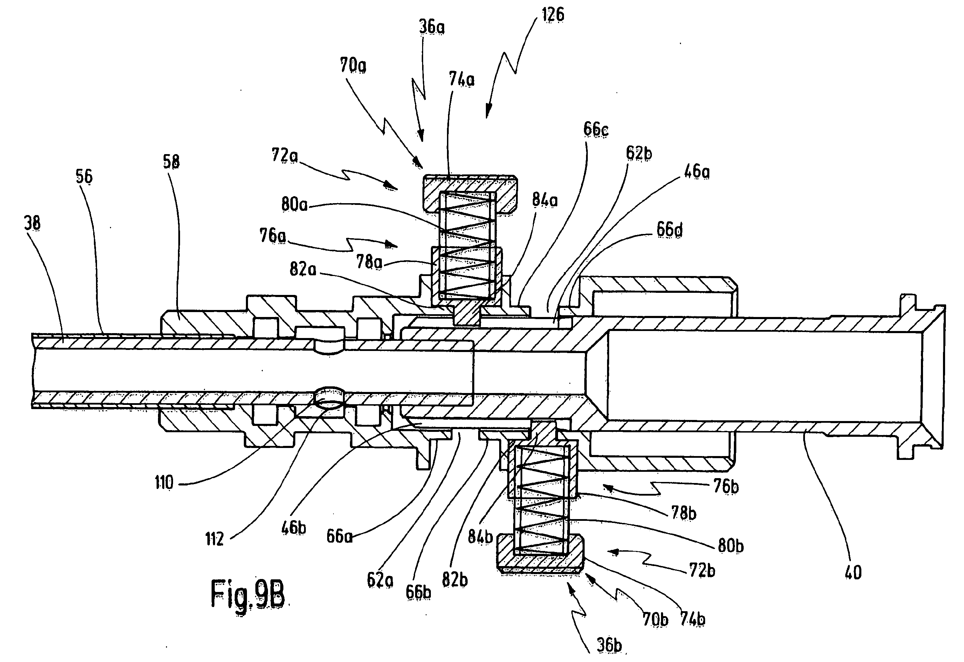

Wird das Handrad 86 beispielsweise in Richtung eines Pfeils 130 gedreht, wird die zweite Drehstellung 126 erreicht, in der Innenschaft 26 relativ zum Außenschaft 28 um einen Winkel von etwa 90° gedreht ist (vgl. Fig. 8B, 9B). In dieser Drehstellung 126 reichen die Stifte 70a, b mit ihren Fortsätzen 84a, b durch die Schlitze 62a, b hindurch und befinden sich beide gleichzeitig mit den Nuten 46a, b in Eingriff. Während der ausgeführten Drehbewegung des Handrads 86 wird der Stift 70a entlang der Außenfläche 109 der Außenrohraufnahme 58 gedreht, bis er ein zu ihm weisendes Schlitzende des Schlitzes 62a erreicht. Aufgrund der abnehmenden radialen Beabstandung der Außenflächen 68a, b der Nocken 66a, b von der Innenrohraufnahme 40 kommt der Stift 70a gerade zum Zeitpunkt der zweiten Drehstellung 126 mit der Nut 46a in Eingriff. Während der Drehbewegung des Handrads 86 von der ersten Drehstellung 124 in die zweite Drehstellung 126 wird der Stift 70b auch in die Richtung des Pfeils 130 mitgedreht, so dass sich der Stift 70b entlang des Schlitzes 62b bewegt. Der Fortsatz 84b des Stifts 70b steht während dieser Drehbewegung mit der Nut 46b in Eingriff und ist noch nicht aufgrund der radial variierenden Ausgestaltung der Nocken 66c, d aus der Nut 46b heraus gehoben. Die Drehbewegung des Innenschafts 26 relativ zum Außenschaft 28 wird dabei durch den Stift 70b vermittelt. Die Stifte 70a, b besitzen in der Drehstellung 126 etwa die gleiche Länge, da die Feder 80a des Stifts 70a im Vergleich zur Drehstellung 124 entspannt ist und der Stift 70b seine Länge im Wesentlichen noch beibehält.

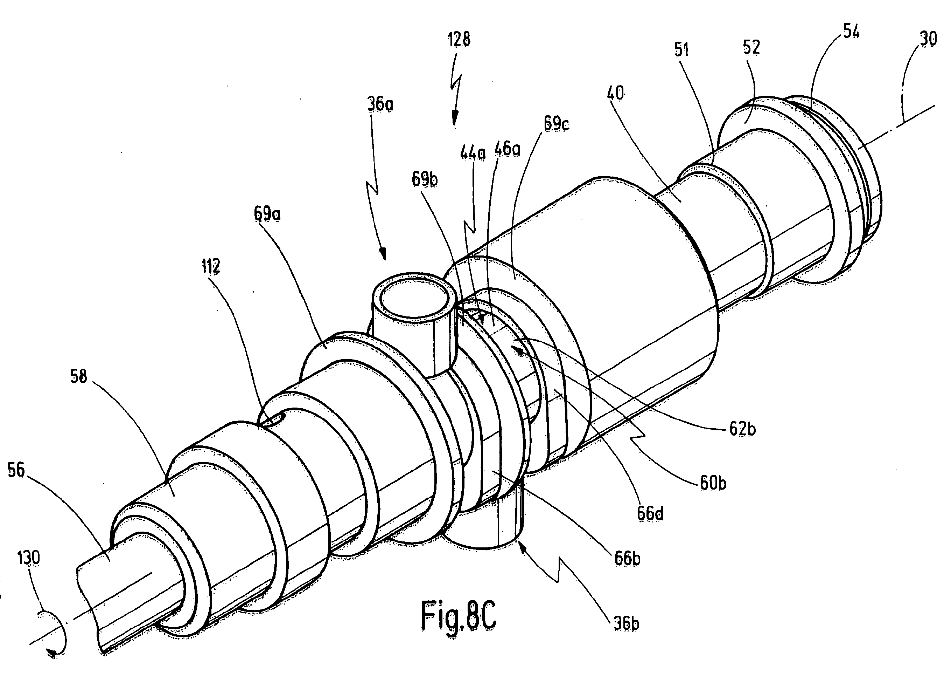

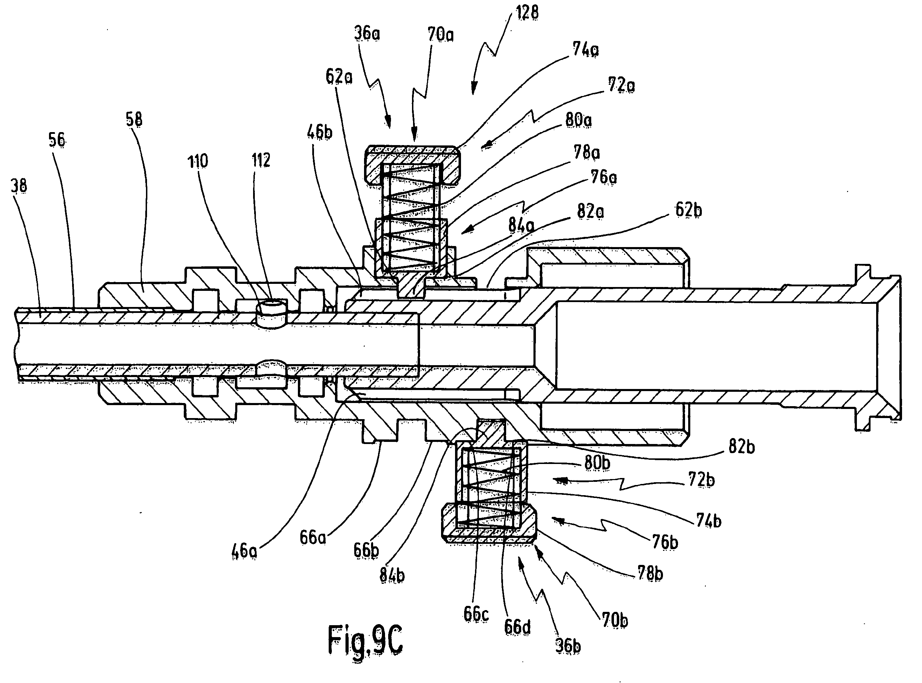

Bei einem Weiterdrehen des Handrads 86 um etwa 90° in Richtung des Pfeils 130 wird die dritte Relativdrehstellung 128 des Innenschafts 26 und des Außenschafts 28 erreicht (vgl. Fig. 8C, 9C). Hierbei kommt der Stift 70a vollständig mit der Nut 46a in Eingriff, während der Stift 70b aus der Nut 46b heraus gehoben wird und an der Außenrohraufnahme 58 außenseitig aufliegt. Der Stift 70a weist in der dritten Drehstellung 128 nun seine maximale Länge auf, während der Stift 70b maximal zusammengedrückt ist.

Jedes Mitnehmerelement 36a, b überträgt folglich eine halbumfängliche Drehung von etwa 180° auf den Innenschaft 26, so dass eine vollumfängliche Relativdrehung des Innenschafts 26 und des Außenschafts 28 um 360° durch zwei Teildrehungen des Innenschafts 26 in die gleiche Drehrichtung bewirkt wird, die jeweils durch eines der zwei Mitnehmerelemente 36a, b übertragenen werden. Aufgrund der symmetrischen Ausgestaltung der Drehübertragungsmechanik 32 kann der Innenschaft 26 relativ zum Außenschaft 28 in beide Drehrichtungen um die Längsachse 30 des medizinischen Instruments 10 gedreht werden.

In einem weiteren Ausführungsbeispiel weist die Innenrohraufnahme 40 nur eine Nut 46a auf. Die Mitnehmerelemente 36a, b sind in Längsrichtung 30 des medizinischen Instruments 10 versetzt und zueinander axial benachbart angeordnet. Dazu sind die Durchbohrungen 88a, b des Handrads 86 in der Längsrichtung 30 versetzt und zueinander axial benachbart angeordnet. Die etwa halbumfänglichen Schlitze 62a, b und die Steuerkurven 64a-d der Außenrohraufnahme 58 sind ferner in der Längsrichtung 30 und umfänglich um etwa 180° zueinander versetzt angeordnet, so dass wiederum die Mitnehmerelemente 36a, b abwechselnd mit der Nut 40 in Eingriff stehen, um die Drehbewegung des Handrads 86 abwechselnd auf die Innenrohraufnahme 40 zu übertragen.

Weist das medizinische Instrument 10 mehr als zwei Mitnehmerelemente 36, beispielsweise n Mitnehmerelemente 36, auf, so wird die Relativdrehung des Innenschafts 26 und des Außenschafts 28 durch jeweils ein mit dem Innenschaft 26 in Eingriff stehendes Mitnehmerelement 36 übertragen. Hierbei können die Mitnehmerelemente 36 umfänglich gesehen um einen Winkel von etwa 360°/n zueinander versetzt angeordnet sein, während die Außenrohraufnahme n axial benachbart angeordnete Öffnungen 60 und die Innenrohraufnahme 40 n umfänglich um einen Winkel von etwa 360°/n zueinander versetzte Aussparungen 44 aufweist. Im Falle, dass die Öffnungen 60 als Schlitze 62 ausgestaltet sind, weisen diese einen Öffnungsbereich von etwa 360°/n auf. Die Steuerkurven 66 der Außenrohraufnahme 58 sind axial benachbart angeordnet und ihre Außenflächen 67 weisen über einen Winkelbereich von etwa 360°(n-1)/n eine derartige radiale Beabstandung von der Innenrohraufnahme 40 auf, dass das jeweilige Mitnehmerelement 36 nur über einen verbleibenden Winkelbereich von etwa 360°/n mit der jeweiligen Aussparung 44 in Eingriff steht.

Es ist ebenfalls möglich, dass die n Mitnehmerelemente 36 in die Längsrichtung 30 des medizinischen Instruments 10 versetzt und axial benachbart angeordnet sind, während die n Öffnungen 60 der Außenrohraufnahme 58 entsprechend in die Längsrichtung 30 und umfänglich gesehen um einen Winkel von etwa 360°/n versetzt angeordnet sind. Die als die Schlitze 62 ausgebildeten Öffnungen 60 weisen hierbei einen Öffnungswinkel von etwa 360°/n auf. Die Innenrohraufnahme 40 weist ferner vorzugsweise nur eine Aussparung 44 auf, in die abwechselnd die Mitnehmerelemente 36 eingreifen. Die Steuerkurven 66 der Außenrohraufnahme 58 sind umfänglich gesehen um einen Winkel von etwa 360°/n zueinander versetzt angeordnet und ihre Außenflächen 67 weisen jeweils über einen Winkelbereich von etwa 360°(n-1)/n eine derartige radiale Beabstandung von der Innenrohraufnahme 40 auf, dass die Mitnehmerelemente 36 in diesem Winkelbereich außer Eingriff mit dem Innenschaft 26 gebracht werden können.

## Patentansprüche

1. Medizinisches Instrument, mit einem Schaft (12), der einen Außenschaft (28) und einen Innenschaft (26) aufweist, wobei der Außenschaft (28) den Innenschaft (26) umgibt, mit einem Stellglied (34) zum Drehen des Innenschafts (26) um eine Längsachse (30) relativ zum Außenschaft (28), wobei das Stellglied (34) an einem proximalen Endbereich (20) des Schafts (12) angeordnet ist, und mit zumindest einem ersten Mitnehmerelement (36a), das mit dem Innenschaft (26) in Eingriff steht, um eine Drehbewegung des Stellglieds (34) auf den Innenschaft (26) zu übertragen, **gekennzeichnet durch** zumindest zwei Mitnehmerelemente (36a, b), die abwechselnd mit dem Innenschaft (26) in Eingriff stehen, um die Drehbewegung des Stellglieds (34) auf den Innenschaft (26) zu übertragen, wobei die Mitnehmerelemente (36a, b) **durch** umfänglich begrenzte und zueinander in der Längsrichtung (30) versetzte Öffnungen (60a, b) in einem proximalen Endbereich (102) des Außenschafts (28) hindurchreichen.

2. Instrument nach Anspruch 1, **dadurch gekennzeichnet, dass** der Außenschaft (28) benachbart zu den Öffnungen (60a, b) des Außenschafts (28) Steuerkurven (64a-d) für die Mitnehmerelemente (36a, b) aufweist, die zum abwechselnden Außer-Eingriff- und In-Eingriff-Bringen der Mitnehmerelemente (36a, b) mit dem Innenschaft (26) dienen.

3. Instrument nach Anspruch 2, **dadurch gekennzeichnet, dass** die Steuerkurven (64a-d) vollumfänglich ausgebildet und in ihren nicht an den Öffnungen (60a, b) anliegenden Abschnitten (68a-d) derart radial vom Innenschaft (26) beabstandet sind, dass die Mitnehmerelemente (36a, b) außer Eingriff mit dem Innenschaft (26) gebracht werden.

4. Instrument nach einem der Ansprüche 1 bis 3, **dadurch gekennzeichnet, dass** die Mitnehmerelemente (36a, b) umfänglich versetzt angeordnet sind.

5. Instrument nach einem der Ansprüche 1 bis 4, **dadurch gekennzeichnet, dass** die Mitnehmerelemente (36a, b) jeweils zweiteilig ausgebildet sind, wobei ein erstes Mitnehmerelementteil (72a, b) und zweites Mitnehmerelementteil (76a, b) der Mitnehmerelemente (36a, b) mit Federkraft beaufschlagbar sind, wobei das erste Mitnehmerelementteil (72a, b) und zweite Mitnehmerelementteil (76a, b) in radialer Richtung des Schafts 12 relativ zueinander beweglich sind.

6. Instrument nach einem der Ansprüche 1 bis 5, **dadurch gekennzeichnet, dass** sich die Mitnehmerelemente (36a, b) bezüglich des Schafts (12) in radialer Richtung erstrecken.

7. Instrument nach einem der Ansprüche 1 bis 6, **dadurch gekennzeichnet, dass** der Innenschaft (26) zumindest eine Aussparung (44a, b) zur Aufnahme der Mitnehmerelemente (36a, b) aufweist.

8. Instrument nach Anspruch 7, **dadurch gekennzeichnet, dass** die Aussparung (44a, b) als in die Längsrichtung (30) verlaufende Nut (46a, b) ausgebildet ist.

9. Instrument nach einem der Ansprüche 1 bis 8, **dadurch gekennzeichnet, dass** die Öffnungen (60a, b) des Außenschafts (28) als Schlitze (62a, b) ausgebildet sind.

10. Instrument nach einem der Ansprüche 1 bis 9, **gekennzeichnet durch** genau zwei Mitnehmerelemente (36a, b), wobei die Öffnungen (60a, b) des Außenschafts (28) etwa halbumfänglich ausgebildet sind.

11. Instrument nach Anspruch 10, **dadurch gekennzeichnet, dass** die zwei Mitnehmerelemente (36a, b) umfänglich um etwa 180° versetzt angeordnet sind.

12. Instrument nach Anspruch 10, **dadurch gekennzeichnet, dass** die zwei Mitnehmerelemente (36a, b) axial benachbart angeordnet sind.

## Claims

1. A medical instrument, comprising a shaft (12), which has an outer shaft (28) and an inner shaft (26), wherein the outer shaft (28) encloses the inner shaft (26), further comprising an actuator (34) for rotating the inner shaft (26) about a longitudinal axis (30) relative to the outer shaft (28), wherein the actuator (34) is arranged at a proximal end region (20) of the shaft (12), and further comprising at least one first tappet element (36a), which is engaged with the inner shaft (26), for transferring a rotary movement of the actuator (34) to the inner shaft (26), **characterized by** at least two tappet elements (36a, b) which are engaged interchangeably with the inner shaft (26) for transferring the rotary movement of the actuator (34) to the inner shaft (26), wherein the tappet elements (36a, b) reach through peripherally limited openings (60a, b) in a proximal end region (102) of the outer shaft (28), which openings are offset with respect to one another in the longitudinal direction (30).

2. The instrument of claim 1, **characterized in that** adjacent to the openings (60a, b) of the outer shaft (28) the outer shaft (28) has control cams (64a-d) for the tappet elements (36a, b), which control cams serve for alternate disengagement and engagement of the tappet elements (36a, b) with the inner shaft (26).

3. The instrument of claim 2, **characterized in that** the control cams (64a-d) are designed such that they have a full circumference and in their sections (68a-d) which are not immediately adjacent to the openings (60a, b) are distanced radially from the inner shaft (26) such that the tappet elements (36a, b) are disengaged from the inner shaft (26).

4. The instrument of any one of claims 1 through 3, **characterized in that** the tappet elements (36a, b) are arranged peripherally offset.

5. The instrument of any one of claims 1 through 4, **characterized in that** the tappet elements (36a, b) are designed in each case in two parts, wherein a first tappet element part (72a, b) and second tappet element part (76a, b) of the tappet elements (36a, b) are supplied with resilient force, wherein the first tappet element part (72a, b) and second tappet element part (76a, b) can be moved in a radial direction of the shaft (12) relative to one another.

6. The instrument of any one of claims 1 through 5, **characterized in that** the tappet elements (36a, b) extend in a radial direction with respect to the shaft (12).

7. The instrument of any one of claims 1 through 6, **characterized in that** the inner shaft (26) has at least one recess (44a, b) for accommodating the tappet elements (36a, b).

8. The instrument of claim 7, **characterized in that** the recess (44a, b) is designed as a groove (46a, b) running in the longitudinal direction (30).

9. The instrument of any one of claims 1 through 8, **characterized in that** the openings (60a, b) of the outer shaft (28) are designed as slots (62a, b).

10. The instrument of any one of claims 1 through 9, **characterized by** exactly two tappet elements (36a, b), wherein the openings (60a, b) of the outer shaft (28) are designed approximately semi-peripherally.

11. The instrument of claim 10, **characterized in that** the two tappet elements (36a, b) are arranged offset peripherally by approximately 180°.

12. The instrument of claim 10, **characterized in that** the two tappet elements (36a, b) are arranged axially adjacent.

## Revendications

1. Instrument médical, comportant une tige (12), qui comprend une tige extérieure (28) et une tige intérieure (26), la tige extérieure (28) entourant la tige intérieure (26), comportant un actionneur (34) destiné à faire tourner la tige intérieure (26) par rapport à la tige extérieure (28) autour d'un axe longitudinal (30), l'actionneur (34) étant agencé au niveau d'une zone d'extrémité proximale (20) de la tige (12), et comportant au moins un premier élément d'entraînement (36a), qui est en prise avec la tige intérieure (26) pour transmettre un mouvement de rotation de l'actionneur (34) sur la tige intérieure (26), **caractérisé par** au moins deux éléments d'entraînement (36a, b) qui entrent en prise en alternance avec la tige intérieure (26) pour transmettre le mouvement de rotation de l'actionneur (34) sur la tige intérieure (26), les éléments d'entraînement (36a, b) passant à travers des ouvertures (60a, b), délimitées sur le pourtour et décalées entre elles dans la direction longitudinale (30), dans une zone d'extrémité proximale (102) de la tige extérieure (28).

2. Instrument selon la revendication 1, **caractérisé en ce que** la tige extérieure (28) comporte, à proximité des ouvertures (60a, b) de la tige extérieure (28), des cames (64a-d) pour les éléments d'entraînement (36a, b), lesquelles sont destinées à amener les éléments d'entraînement (36a, b) en alternance en prise et hors de prise avec la tige intérieure (26).

3. Instrument selon la revendication 2, **caractérisé en ce que** les cames (64a-d) sont réalisées sur un pourtour complet et, dans leurs zones (68a-d) en appui contre les ouvertures (60a, b), sont situées à une distance radiale de la tige intérieure (26), de telle sorte que les éléments d'entraînement (36a, b) sont amenés hors de prise avec la tige intérieure (26).

4. Instrument selon l'une quelconque des revendications 1 à 3, **caractérisé en ce que** les éléments d'entraînement (36a, b) sont disposés en étant décalés sur le pourtour.

5. Instrument selon l'une quelconque des revendications 1 à 4, **caractérisé en ce que** les éléments d'entraînement (36a, b) sont réalisés chacun en deux parties, une première partie (72a, b) et une deuxième partie (76a, b) des éléments d'entraînement (36a, b) pouvant être soumises à une force de ressort, ladite première partie (72a, b) et ladite deuxième partie (76a, b) étant mobiles l'une par rapport à l'autre dans la direction radiale de la tige (12).

6. Instrument selon l'une quelconque des revendications 1 à 5, **caractérisé en ce que** les éléments d'entraînement (36a, b) s'étendent dans la direction radiale par rapport à la tige (12).

7. Instrument selon l'une quelconque des revendications 1 à 6, **caractérisé en ce que** la tige intérieure (26) comporte au moins un évidement (44a, b) destiné à recevoir les éléments d'entraînement (36a, b).

8. Instrument selon la revendication 7, **caractérisé en ce que** l'évidement (44a, b) est réalisé sous la forme d'une rainure (46a, b) orientée dans la direction longitudinale (30).

9. Instrument selon l'une quelconque des revendications 1 à 8, **caractérisé en ce que** les ouvertures (60a, b) de la tige extérieure (28) sont réalisées sous forme de fentes (62a, b).

10. Instrument selon l'une quelconque des revendications 1 à 9, **caractérisé par** exactement deux éléments d'entraînement (36a, b), les ouvertures (60a, b) de la tige extérieure (28) étant réalisées sensiblement sur une moitié du pourtour.

11. Instrument selon la revendication 10, **caractérisé en ce que** les deux éléments d'entraînement (36a, b) sont disposés en étant décalés de 180° environ sur le pourtour.

12. Instrument selon la revendication 10, **caractérisé en ce que** les deux éléments d'entraînement (36a, b) sont disposés en étant axialement adjacents.
